Europäisches Patentamt

European Patent Office

Office européen des brevets

(19)

(11) Veröffentlichungsnummer: **0 157 235**
**A1**

(12) # EUROPÄISCHE PATENTANMELDUNG

(21) Anmeldenummer: **85102802.7**

(22) Anmeldetag: **12.03.85**

(51) Int. Cl.⁴: **C 12 N  15/00,** C 12 P  21/00,
C 07 K  3/12, C 07 K  3/28,
C 07 K  17/00, C 12 N  9/00

(30) Priorität: **22.03.84  DE 3410437**

(43) Veröffentlichungstag der Anmeldung: **09.10.85**
**Patentblatt 85/41**

(84) Benannte Vertragsstaaten: **AT BE CH DE FR GB IT LI NL SE**

(71) Anmelder: **BAYER AG, Konzernverwaltung RP
Patentabteilung, D-5090 Leverkusen 1 Bayerwerk (DE)**

(72) Erfinder: **Schmidt-Kastner, Günter, Prof. Dr.,
Falkenberg 59, D-5600 Wuppertal 1 (DE)**
Erfinder: **Kutzbach, Carl, Dr., Miles Laboratories,
Inc. 1127 Myrtle Street, Elkhart Indiana 46514 (US)**

(54) **Verfahren zur Herstellung von Proteinen.**

(57)    Die Erfindung betrifft ein Verfahren zur Herstellung von Proteinen durch Co-Clonierung von DNA-Sequenzen für ein gewünschtes Protein mit solchen für zwei bestimmte weitere Peptide mittels gentechnologischer Methoden unter Expression eines kombinierten Proteins aus den drei Bestandteilen. Dieses kombinierte Protein wird dann biospezifisch an eine trägergebundene, biospezifisch-komplementäre Substanz adsorbiert und nach Abtrennung von übrigen Bestandteilen so gespalten, daß nur wenige Bruchstücke vorliegen, von denen eines das gewünschte Protein ist.

BAYER AKTIENGESELLSCHAFT          5090 Leverkusen, Bayerwerk

Konzernverwaltung RP              Dn/by-c
Patentabteilung


Verfahren zur Herstellung von Proteinen

Die Erfindung betrifft ein Verfahren zur Herstellung von Proteinen durch Co-Clonierung von DNA-Sequenzen für ein gewünschtes Protein mit solchen für zwei bestimmte weitere Peptide mittels gentechnologischer Methoden unter Expression eines kombinierten Proteins aus den drei Bestandteilen. Dieses kombinierte Protein wird dann biospezifisch an eine trägergebundene, biospezifisch-komplementäre Substanz adsorbiert und nach Abtrennung von übrigen Bestandteilen so gespalten, daß nur wenige Bruchstücke vorliegen, von denen eines das gewünschte Protein ist.

Bei der gentechnologischen Herstellung von Proteinen ist es stets ein Problem, das exprimierte Protein aus einem Vielstoff-Gemisch in reiner Form zu isolieren.

Die Erfindung stellt nun ein konzeptionell neues Verfahren zur gentechnologischen Herstellung und Gewinnung eines Proteins zur Verfügung.

Le A 22 976-Ausland

Dieses neue Verfahren besteht darin, daß man

a)  eine DNA-Sequenz, die für dieses Protein codiert,
eine DNA-Sequenz, die für ein kurzkettiges Peptid
codiert und
eine DNA-Sequenz, die für ein biospezifisches Polypeptid codiert
derart miteinander verknüpft, daß sich die DNA-
Sequenz für das kurzkettige Peptid zwischen den
beiden anderen DNA-Sequenzen befindet;

b)  das so erhaltene neue DNA-Stück mittels geeigneter
gentechnologischer Methoden so in die DNA eines
Wirtsorganismus einbringt, daß Transkription,
Translation und Expression eines kombinierten
Proteins erfolgen können, das aus dem biospezifischen Polypeptid, dem kurzkettigen Peptid
und dem gewünschten Protein besteht;

c)  den Wirtsorganismus so kultiviert, daß er das kombinierte Protein erzeugt;

d)  den das kombinierte Protein enthaltenden Teil des
Kultivierungssystems, gegebenenfalls nach Abtrennen
anderer Bestandteile, mit einem immobilisierten
System in Kontakt bringt, das aus einem geeigneten
Trägermaterial besteht, mit dem eine Substanz verbunden ist, die zu dem biospezifischen Polypeptid
komplementär ist und mit ihm unter den gegebenen
Reaktionsbedingungen eine starke Bindung eingeht;

e)   das immobilisierte System, an das das kombinierte
     Protein gebunden hat, in geeigneter Weise von
     übrigen Bestandteilen trennt und mit einem Mittel
     behandelt, das spezifisch das kurzkettige Peptid
     aus dem gebundenen kombinierten Protein abspaltet
     und

f)   das auf diese Weise freigesetzte gewünschte Protein
     in geeigneter Weise von dem übrigen Bestandteilen
     trennt und gewinnt.

Das Verfahren der Erfindung beinhaltet somit die Maßnahme, das gewünschte Protein nicht isoliert exprimieren
zu lassen, sondern verbunden mit zwei anderen Elementen,
dem biospezifischen Polypeptid und dem kurzkettigen
Peptid.

In diesem Konzept stellt das kurzkettige Peptid, das
zwischen dem gewünschten Protein und dem biospezifischen
Polypeptid angeordnet ist, die Sollbruchstelle dar.
Das kurzkettige Peptid muß daher so beschaffen sein,
daß es im späteren Verfahrensgang hochspezifisch herausgeschnitten werden kann. Dieses Herausschneiden erfolgt
vorzugsweise mit einem Enzym. Die Wahl des kurzkettigen
Peptids richtet sich somit danach, ob ein Mittel, beispielsweise ein Enzym, zur Verfügung steht, das in der
Lage ist, die Aminosäure-Sequenz des kurzkettigen
Peptids zu erkennen und zu spalten. Es genügt aber auch
eine nur einseitige Spaltung der Bindung zwischen dem
kurzkettigen Peptid und dem gewünschten Protein.

Le A 22 976

Ein solches hochspezifisches Enzym ist z.B. das aus Pankreas gewonnene Enzym Kallikrein (E.C. 3.4.21.8.). Dieses Enzym spaltet aus der Peptidkette des Kininogens die Sequenz des Dekapeptids Lys-Arg-Pro-Pro-Gly-Phe-Ser-Pro-Phe-Arg heraus. Dieses Dekapeptid wird erfindungsgemäß bevorzugt co-cloniert.

Das weiterhin erfindungsgemäß co-clonierte biospezifische Polypeptid hat die Aufgabe, an eine trägergebundene, biospezifisch-komplementäre Substanz zu binden, damit der Träger zusammen mit dem daran gebundenen kombinierten Protein von übrigen Bestandteilen des Mediums getrennt werden kann. Daraus folgt, daß an das System biospezifisches Polypeptid/komplementäre Substanz die Anforderung zu stellen ist, daß unter den gegebenen Reaktionsbedingungen eine starke Bindung zwischen ihnen erfolgt.

Biologisch aktive Polypeptide, z.B. Enzyme, können biospezifisch mit biologisch wirksamen komplementären Substanzen, z.B. mit Inhibitoren Komplexe, z.B. Enzym-Inhibitor-Komplexe bilden.

Solche biospezifisch gebildeten Enzym-Inhibitor-Komplexe besitzen eine hohe Bindungsaffinität. Ihre Dissoziationskonstante ist dementsprechend klein.

Ein Beispiel für einen Enzym-Inhibitor-Komplex ist der erfindungsgemäß bevorzugte Trypsin-Aprotinin-Komplex, der aus dem Enzym Trypsin und aus dem Enzyminhibitor

Le A 22 976

Aprotinin gebildet wird. Die Dissoziationskonstante Ki dieses Komplexes liegt bei nur $6 \cdot 10^{-14}$ mol/l (pH = 8,0, t = 25°) die Komplexbildungsgeschwindigkeit bei t 1/2 = 6,3 Sekunden. Der Trypsin-Aprotinin-Komplex wird dementsprechend sehr schnell gebildet und besitzt eine sehr hohe Stabilität unter den gegebenen Bedingungen. Die Stabilität des Trypsin-Aprotinin-Komplexes ist jedoch abhängig vom pH-Wert der Lösung und nimmt ab mit sinkendem pH-Wert. Bei einem pH-Wert von ca. 2,0 ist der Trypsin-Aprotinin-Komplex fast vollständig dissoziiert.

Ein solches Verhalten ist im Rahmen der Erfindung besonders wünschenswert, weil hierüber leicht eine Regenerierung der trägergebundenen Komplementärsubstanz möglich ist. Diese kann dann erneut im Verfahren eingesetzt werden. Es ist ferner wünschenswert, daß die Bildungsgeschwindigkeit der Bindung zwischen dem biospezifischen Polypeptid und der trägergebundenen Komplementärsubstanz hoch ist. Auf diese Weise lassen sich mögliche Konkurrenzreaktionen der trägergebundenen Substanz mit anderen Stellen des kombinierten Polypeptids reduzieren.

Das geschilderte System Enzym/Inhibitor ist lediglich beispielhaft. Ebenso können beispielsweise eingesetzt werden die Systeme Antigen/Antikörper oder Peptidhormon/Rezeptor.

Le A 22 976

Die biospezifische Adsorption erfolgt bevorzugt unter Verwendung von in Wasser unlöslichen Trägern, an die die biospezifisch-komplementäre Substanz gebunden ist. Diese Bindung kann nach den bekannten Methoden der Immobilisierung mit oder ohne Spacer-Moleküle erfolgen.

Die DNA-Sequenz der drei genannten Komponenten werden nach Methoden der Gentechnologie co-cloniert und die so erhaltene DNA nach Methoden der Gentechnologie in die DNA eines Wirtsorganismus, z.B. in ein Plasmid, übertragen. Als Wirtsorganismus können geeignete Mikroorganismen genutzt werden, z.B. Bakterien, insbesondere E. coli, aber auch Bac. subtilis oder Hefen. Die Transkription, Translation und letztlich die Expression der co-clonierten DNA erfolgt während der Kultivierung des Mikroorganismus. Ist das gewünschte Protein mit den beiden co-clonierten Peptiden in der Fermentationslösung enthalten, so werden die Zellen, z.B. durch Zentrifugieren oder durch Cross-flow-Filtration abgetrennt und das Kulturfiltrat zur Isolierung verwendet. Ist das gewünschte Protein mit den beiden co-clonierten Peptiden in der Zelle selbst enthalten, so werden die Zellen separiert, die Zellen aufgeschlossen und die Zellbruchstücke durch Separation abgetrennt. Das Kulturfiltrat oder der Zellextrakt enthalten nicht nur das gewünschte co-clonierte kombinierte Protein, sondern darüber hinaus eine Vielzahl weiterer Proteine, eine Vielzahl anderer Produkte, aber auch anhaftende Nährmediumbestandteile. Aus dieser komplex zusammengesetzten Lösung wird nun das gewünschte kombinierte Protein isoliert und gereinigt. Dies erfolgt dadurch, daß man die trägergebundene biospezifisch-

Le A 22 976

komplementäre Substanz, z.B. das immobilisierte Trypsin zu dem Kulturfiltrat oder Extrakt gibt. Es bildet sich augenblicklich mit hoher Komplexbildungsgeschwindigkeit der wasserunlösliche Komplex aus den fünf Komponenten: Träger + biospezifisch-komplementäre Substanz + biospezifisches Polypeptid + kurzkettiges Peptid + gewünschtes Protein, z.B. der Komplex aus den fünf Komponenten Träger + Trypsin + Aprotinin + Dekapeptid + gewünschtes Protein. Die Zugabe der carriergebundenen biospezifisch-komplementären Substanz kann bei Zimmertemperatur, aber auch, bei der hohen Komplexbildungsgeschwindigkeit, bei niedrigen Temperaturen, z.B. bei + 2°C erfolgen. Der wasserunlösliche Komplex kann von anderen Proteinen der Fermentationslösung und auch von allen anderen Verunreinigungen durch einfache Filtration abgetrennt werden.

Wird als biospezifisch-komplementäre Substanz ein proteolytisches Enzym, z.B. Trypsin, verwendet, so kann dieses vor der Zugabe zur Fermentationslösung mit einem niedermolekularen Inhibitor abgesättigt werden. Die Dissoziationskonstante des niedermolekularen Inhibitors muß jedoch größer als die des biospezifischen Polypeptids sein. Der niedermolekulare Inhibitor wird auf Grund der unterschiedlichen Dissoziationskonstanten bei Zugabe der Fermentationslösung von dem biospezifischen Polypeptid verdrängt. Als niedermolekularer Inhibitor für das trägergebundene Trypsin kann z.B. Benzamidin mit einer Dissoziationskonstante von $18.10^{-6}$ mol/l oder Phenylguanidin mit einer Dissoziationskonstante von $72 . 10^{-6}$ mol/l verwendet werden.

Le A 22 976

Alternativ kann die trägergebundene, biospezifisch-
komplementäre Substanz in eine Säule gefüllt werden.
Durch die Säule wird dann das Kulturfiltrat oder der
Zellextrakt gegeben. Auch hier wird ausschließlich
das gewünschte kombinierte Protein über das biospezifische Polypeptid an den festen Träger gebunden, während alle anderen Proteine und alle anderen
Verunreinigungen durch die Säule laufen. Das an den
Carrier gebundene biospezifisch-komplementäre Polypeptid sollte möglichst vollständig mit dem zu bindenden Komplex abgesättigt sein. Die Säule kann durch
Waschen mit Pufferlösungen oder durch Waschen mit anderen
geeigneten Lösungen von restlichen, anhaftenden Verunreinigungen befreit werden. Bei einer hohen Komplexbildungsgeschwindigkeit kann die Säule auch bei tiefen
Temperaturen beschickt werden.

Schließlich wird durch Einwirkung eines spezifischen
Enzyms das am Träger gebundene kombinierte Protein
aufgespalten, wobei das spezifische Enzym ausschließlich die Peptidbindungen des kurzkettigen Peptides
spaltet.

Ist der Gesamtkomplex aus den Komponenten Träger +
Trypsin + Aprotinin + Dekapeptid + gewünschtes Protein
aufgebaut, so kann, wie erwähnt, als spezifisches
Enzym das Enzym Kallikrein verwendet werden. Das
Kallikrein hydrolysiert beidseitig das Dekapeptid und
löst das endständig gebundene, gewünschte Protein vom
kombinierten Protein. Es genügt aber auch nur eine
einseitige Spaltung der Bindung zwischen einem kurzkettigen Peptid und dem gewünschten Protein. Ein solches kurzkettiges Peptid könnte z. B. nur die letzten drei C-terminalen Aminosäuren des obengenannten Dekapeptids mit der
Sequenz Pro-Phe-Arg enthalten. So spaltet das Kallikrein

Le A 22 976

hochspezifisch an der gewünschten Sollbruchstelle
...Arg-X... die Hexpeptide:

$$\text{Pro-PheArg-Ser-Tyr-Gln} \qquad K_M = 0,25 \text{ m Mol}$$
$$\text{oder} \qquad \text{Pro-Phe-Arg-Ala-Asn-Leu} \qquad K_M = 0,15 \text{ m Mol.}$$

Das gewünschte Protein wird zusammen mit dem Dekapeptid eluiert und kann vom Dekapeptid nach bekannten Methoden, z.B. durch Molekularsiebchromatographie, abgetrennt werden. Das gewünschte Protein wird so in reiner Form erhalten. Der Vorzug in der Wahl eines kurzkettigen Peptids als Sollbruchstelle im kombinierten Protein ist darin zu sehen, daß nach der Spaltung das gewünschte Protein als in der Regel großes Molekül von dem kleinen kurzkettigen Peptid leicht trennbar ist.

Das biospezifische Polypeptid bleibt über die komplementäre Substanz unter den Bedingungen der Elution am Träger gebunden. Erst durch Wechsel z.B. des pH-Wertes einer Pufferlösung, z.B. auf einen Wert von 2-3, wird das biospezifische Polypeptid abgelöst und aus der Säule ausgewaschen. Die Säule kann dann nach weiteren Waschvorgängen wieder mit Pufferlösungen auf einen geeigneten pH-Wert, bei Verwendung von Trypsin/Aprotinin auf pH 8,0 eingestellt und für einen weiteren Versuch wiederverwendet werden.

Als Proteine, die nach dem erfindungsgemäßen Verfahren hergestellt werden können, sind u.a. Polypeptidsequenzen von Hormonen, z.B. des Human-Somatotropins, des Erythropoitins, des Corticotropins, des Pre-pro-Insulins, des Pre-minipro-Insulins, der A- und B-Kette des Insulins, Polypeptidsequenzen von Neutrotransmitter, z.B. der Substanz P oder des ß-Endomorphins, Polypeptidsequenzen der Interferone, des INF-$\alpha$, INF-ß, INF-$\gamma$, der Hybridinterferone, der Interleukine, Polypeptidsequenzen von Plasmaproteinen, z.B. des Human-$\alpha_1$-

Antitrypsins, des Albumins, der Gerinnungsfaktoren, z.B. des Tissue-Plasminogen-Aktivators, des Faktors VIII, Polypeptidsequenzen von Enzymen, z.B. der D-Xylose-Isomerase, der Penicillinacylase, der Urokinase, des Rennins, Polypeptidsequenzen von Antikörpern, z.B. von monoclonalen Antikörpern und von Vakzinen zu nennen.

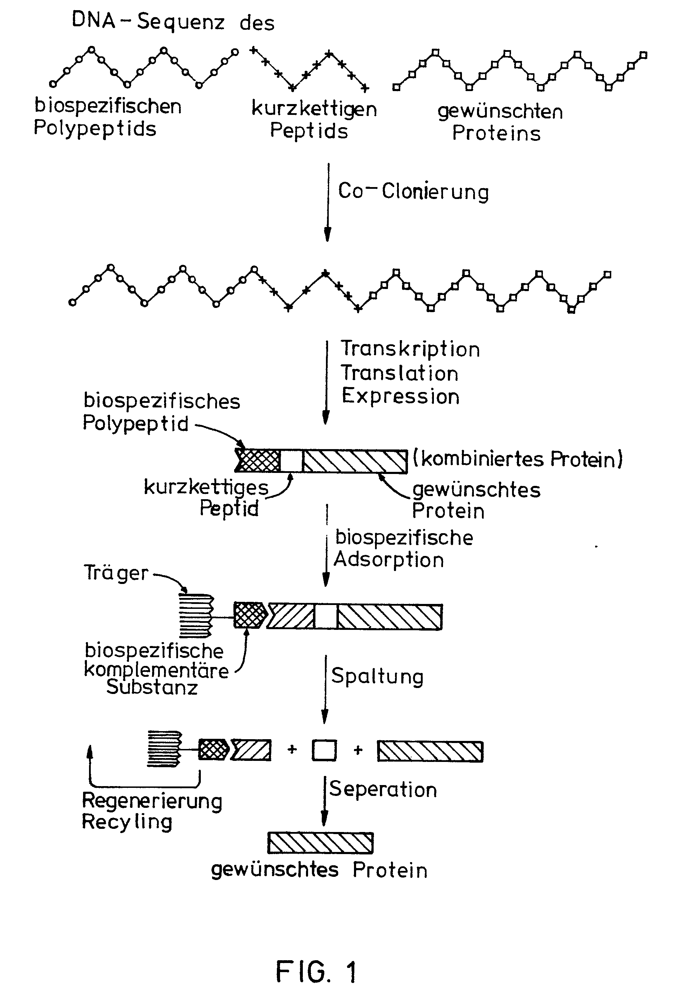

Die Abbildung 1 veranschaulicht das erfindungsgemäße Verfahren in schematisierter Form.

Le A 22 976

<u>Patentansprüche</u>

1.  Verfahren zur Herstellung eines Proteins, dadurch
    gekennzeichnet, daß man

    a)  eine DNA-Sequenz, die für dieses Protein
        codiert,
        eine DNA-Sequenz, die für ein kurzkettiges
        Peptid codiert und
        eine DNA-Sequenz, die für ein biospezifisches
        Polypeptid codiert
        derart miteinander verknüpft, daß sich die DNA-
        Sequenz für das kurzkettige Peptid zwischen den
        beiden anderen DNA-Sequenzen befindet;

    b)  das so erhaltene neue DNA-Stück mittels geeigneter gentechnologischer Methoden so in
        die DNA eines Wirtsorganismus einbringt, daß
        Transkription, Translation und Expression eines
        kombinierten Proteins erfolgen können, das aus
        dem bisspezifischen Polypeptid, dem kurzkettigen
        Peptid und dem gewünschten Protein besteht;

    c)  den Wirtsorganismus so kultiviert, daß er das
        kombinierte Protein erzeugt;

    d)  den das kombinierte Protein enthaltenden Teil des
        Kultivierungssystems, gegebenenfalls nach Abtrennen
        anderer Bestandteile, mit einem immobilisierten
        System in Kontakt bringt, das aus einem geeigneten

<u>Le A 22 976</u>

0157235

Trägermaterial besteht, mit dem eine Substanz verbunden ist, die zu dem biospezifischen Polypeptid
komplementär ist und mit ihm unter den gegebenen
Reaktionsbedingungen eine starke Bindung eingeht;

e)   das immobilisierte System, an das das kombinierte
     Protein gebunden hat, in geeigneter Weise von
     übrigen Bestandteilen trennt und mit einem Mittel
     behandelt, das spezifisch das kurzkettige Peptid
     aus dem gebundenen kombinierten Protein spaltet, und

f)   das auf diese Weise freigesetzte gewünschte
     Protein in geeigneter Weise von dem übrigen
     Bestandteilen trennt und gewinnt.

2.   Verfahren nach Anspruch 1, dadurch gekennzeichnet,
     daß das biospezifische Polypeptid mit der trägergebundenen, zu ihr komplementären Substanz mit
     hoher Bildungsgeschwindigkeit die starke Bindung
     eingeht.

3.   Verfahren nach Anspruch 2, dadurch gekennzeichnet,
     daß die Bindung bei Temperaturen von + 2°C bis Raumtem
     peratur erfolgt.

4.   Verfahren nach den Ansprüchen 1 bis 3, dadurch
     gekennzeichnet, daß das biospezifische Polypeptid
     und die zu ihm komplementäre trägergebundene
     Substanz ein System aus Enzym und dazugehörigem
     Inhibitor darstellen.

Le A 22 976

5. Verfahren nach den Ansprüchen 1 bis 4, dadurch gekennzeichnet, daß das biospezifische Polypeptid Aprotinin und die zu ihm komplementäre trägergebundene Substanz Trypsin ist.

6. Verfahren nach den Ansprüchen 1 bis 5, dadurch gekennzeichnet, daß das kurzkettige Peptid das Dekapeptid Lys-Arg-Pro-Pro-Gly-Phe-Ser-Pro-Phe-Arg ist.

7. Verfahren nach den Ansprüchen 1 bis 6, dadurch gekennzeichnet, daß das Mittel, das das kurzkettige Peptid spezifisch aus dem gebundenen kombinierten Protein spaltet, ein Enzym ist.

8. Verfahren nach den Ansprüchen 6 und 7, dadurch gekennzeichnet, daß das Enzym Kallikrein (E.C.3.4.21.8.) ist.

9. Verfahren nach den Ansprüchen 1 bis 8, dadurch gekennzeichnet, daß das herzustellende Protein ein Hormon, ein Plasmaprotein, ein Gerinnungsfaktor, ein Enzym, ein Antikörper oder eine Vakzine ist.

10. Verfahren nach Anspruch 9, dadurch gekennzeichnet, daß das Protein Faktor VIII ist.

<u>Le A 22 976</u>

DNA-Sequenz des

biospezifischen
Polypeptids

kurzkettigen
Peptids

gewünschten
Proteins

Co-Clonierung

Transkription
Translation
Expression

biospezifisches
Polypeptid

(kombiniertes Protein)

kurzkettiges
Peptid

gewünschtes
Protein

biospezifische
Adsorption

Träger

biospezifische
komplementäre
Substanz

Spaltung

Regenerierung
Recyling

Seperation

gewünschtes Protein

FIG. 1

**0157235**
Nummer der Anmeldung

Europäisches
Patentamt

# EUROPÄISCHER RECHERCHENBERICHT

| EINSCHLÄGIGE DOKUMENTE | | | EP 85102802.7 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl.4)** |
| A | EP - A1 - 0 009 930 (THE BOARD OF TRUSTEES OF THE LELAND STANFORD JUNIOR UNIVERSITY)<br><br>* Zusammenfassung *<br><br>---- | 1 | C 12 N 15/00<br>C 12 P 21/00<br>C 07 K 3/12<br>C 07 K 3/28<br>C 07 K 17/00<br>C 12 N 9/00 |
| | | | **RECHERCHIERTE SACHGEBIETE (Int. Cl.4)**<br><br>C 12 N<br>C 12 P<br>C 07 K<br>A 61 K |
| Der vorliegende Recherchenbericht wurde für alle Patentansprüche erstellt. | | | |

| Recherchenort | Abschlußdatum der Recherche | Prüfer |
|---|---|---|
| WIEN | 28-06-1985 | WOLF |